# EUROPEAN PATENT APPLICATION

(11) **EP 4 285 942 A1**
(43) Date of publication of application: **06.12.2023**
(21) Application number: 22177233.8
(22) Date of filing: 03.06.2022
(51) Int. Cl.: A61L 15/12, A61L 15/26

(54) **THERMOPLASTIC MATERIAL FOR IMMOBILIZATION PURPOSES**

(71) Applicant: Orfit Industries N.V., 2110 Wijnegem (BE)
(72) Inventor: CUYPERS, Steven, 2110 Wijnegem (BE); HUSSEIN, Naji, 2110 Wijnegem (BE)
(74) Representative: V.O.

(57) **Abstract**

A composition for use in an immobilization device for immobilizing at least a portion of a body part comprising, based on total weight of the composition,
a. 30 to 62 wt% of a first polymer with a melting temperature below 100°C, preferably between 40 and 75°C;
b. 15 to 35 wt% of a spherical filler;
c. 4 to 20 wt% of an elastomeric material;
d. optionally 2 to 20 wt% of a second polymer with a higher bending modulus than the first polymer;
e. 0 to 7 wt% of total additives.

## Description

The invention relates to a composition and thermoplastic composite material for use in an immobilization device for immobilizing at least a portion of a human or animal body part. The invention furthermore relates to a method for manufacturing such composite material and immobilization device.

The use of fixation and immobilization devices for immobilizing a part of a body has become well known technology in applications such as orthotics and prosthetics, physical rehabilitation, radiation oncology and diagnostic imaging. Those applications, in particular physical rehabilitation, require that the immobilization device is mouldable at activation temperature directly on a patient, that the immobilization device shows good mechanical properties and surface finishing and is light and comfortable to the patient. Important applications for immobilization devices include physical rehabilitation applications and orthopedic applications for example in splints and braces, to immobilize and protect inflamed or injured joints, to support and immobilize ligaments and fractures and muscular structures and podiatry for example as insole (foot-bed) applications.

To produce fixation or immobilization devices, which are suitable for use in the above described applications, usually use is made of a sheet shaped thermoplastic material, which is moulded to conform as good as possible to the body part that is to be immobilized. Over the years, continuous development has been going on towards materials, which meet specific requirements of the envisaged application. The use of sheets of thermoplastic materials which may be directly moulded to the body part to be immobilized has achieved significant attention, as this permits achieving immobilization with the highest accuracy, where the size and shape of the immobilization device may be directly adapted to each individual patient in the position in which the body part is to be immobilized, and it may be adapted in the course of time by re-moulding the immobilization device. To permit this direct moulding, the thermoplastic material should have a melting temperature which is sufficiently low to be sustained by the body. Besides that, the material should have sufficient formability and elasticity in the molten state, for a period of time which is sufficiently long to permit moulding, but not too long to save clinical time and minimize the risk to deformation after moulding has been completed. A thermoplastic material which is particularly suitable for direct moulding to a body part is commonly based on polycaprolactone. In practice, a polycaprolactone based sheet is heated in a warm water bath, for a period of time sufficiently long to achieve softening and melting of the material, the sheet is then applied to the body part to be immobilized, shaped to conform to the body part and allowed to cool.

Not only a good conformability is important for immobilization devices. Another important feature, in particular for applications where a high rigidity is required such as for example in anti-spasticity orthosis, dorsal blocking orthosis and wrist cock-up, is that such devices have a high bending modulus. The material should have a bending modulus which is sufficiently high to minimize the risk to deformation of the device and the ability of the body part to move. The material should also provide a high stability during use of the immobilization device, i.e. the restriction to move the body parts with respect to the immobilisation device, when immobilised by the immobilisation device. The higher the stability, the more difficult it will be to move the body parts in the immobilised condition.

To obtain a immobilization device made of a thermoplastic material with a high rigidity, attempts have been made to reinforce thermoplastic materials with short glass fibers, which could offer the possibility to reach high level of bending modulus, such as described in WO 2020/006615 and US 5584800. However, extrusion of reinforced glass fibers thermoplastic materials results in sheets with anisotropic mechanical properties. This phenomena is due to the orientation of the glass fibers in the extrusion flow direction. Consequently, the resistance to stretch in cross direction is significantly different from that in extrusion direction.

To avoid such problem, other attempts were directed to the production of reinforced thermoplastic sheets by injection moulding instead of extrusion where the glass fiber orientation is minimal, resulting in a more isotropic product. However production by extrusion is preferred over injection molding since it is a continuous process and more cost effective and has a higher output.

There is thus a need for a thermoplastic material for an immobilization device, which provides a high modulus as well as a good conformability. Furthermore, such immobilization devices should provide a good patient comfort and should not be made of itching materials. Further preferably said thermoplastic material can be produced via extrusion.

It was found that a novel composition could be used to produce a thermoplastic sheet having a high rigidity, without impacting other properties such as the easiness to mould or shape, drape, conformity and easiness to finish the edges.

Accordingly the invention provides a composition comprising, based on total weight of the composition,
a. 30 to 62 wt% of a first polymer with a melting temperature of below 100°C, preferably of between 40 and 75°C;
b. 15 to 35 wt% of a spherical filler and optionally up to 10 wt% of another type of filler;
c. 4 to 20 wt% of an elastomeric material;
d. optionally, 2 to 20 wt% of a second polymer with a higher bending modulus than the first polymer;
e. 0 to 7 wt% of total additives.

The composition according to the invention through careful selection of its components and amounts thereof allows to produce, inter alia through an extrusion process, a thermoplastic composite material suitable as immobilization device that can be easily molded or shaped around the body member. The product in molden state has a high drape behaviour (it is soft so it easily flows and conforms perfectly to the shape of the body member) while it becomes sufficiently rigid once it cools down to room temperature. Despite their relatively high rigidity/stiffness, the orthoses and splints made thereof are highly comfortable.

The composition according to the invention was tuned in such a manner that the material does not cooldown or solidify quickly and remains elastomeric and moldable thereby offering the therapist sufficient time to re-shape and finish the orthosis.

For the purpose of clarity and a concise description, features are described herein as part of the same or separate embodiments, however, it will be appreciated that the scope of the invention may include embodiments having combinations of all or some of the features described.

As used herein, the singular forms "a", "an" and "the" are intended to include the plural forms as well. The term " or" includes any and all combinations of one or more of the associated listed items, unless the context clearly indicates otherwise (e.g. if an "either ....or" construction is used). It will be understood that the terms "comprises" and "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise

As is used herein, the term "wt %", or "weight percentage", or "percentage by weight", refers to the mass fraction of a substance in a mixture divided by the total mass of said mixture, expressed as a percentage between 0 and 100.

As is used herein, the term "thermoplastic composite material", refers to a material composed of two or more connecting materials, and contains a matrix made of a thermoplastic polymer. Usually, a thermoplastic composite material has different material properties from its individual components.

As is used herein, the term "drape", refers to the ability of a material, once activated (i.e. heated to a target temperature e.g. 65°C), to bend about and conform to the surface of a curved article. The ability of a material to drape on the surface of an adherend may be characterized by its bending radius, i.e., the minimum radius about which the material may be bent without kinking, folding, or cracking. Alternatively, the ability of a material to drape may be characterized by measuring the distance between two edges of the material when bended over a curved article such as a metal pin.

The invention provides a composition comprising at least a first polymer, a spherical filler, an elastomeric material and optionally, a second polymer. Said composition may further comprise additives.

A first polymer as comprised in a composition according to the invention is a thermoplastic polymer with a low melting temperature (generally below 100°C or even below 75°C). Preferably the melting temperature of said first polymer is between 40 and 75°C, more preferably between 50 and 70°C, most preferably between 55 and 65°C, as these temperatures may be supported by the human and animal body and permit direct molding to the body. Examples of thermoplastic polymers with such melting temperatures are thermoplastic polyurethane, thermoplastic polyesters and thermoplastic polyolefins. Only one of said polymers can be used or a blend of two or more of them. Examples of suitable thermoplastic polyolefins include ethylene copolymers, ethylene vinyl acetate copolymers, propylene copolymers, ethylene-propylene co-polymers or poly cis-isoprene. Examples of suitable thermoplastic polyesters include polycaprolactone, polymeric fatty acid esters, butylene adipate terephthalate copolymers, succinate-butylene adipate copolymers. Preferred first polymers are selected from the group consisting of polycaprolactone (PCL), thermoplastic polyurethane (TPU), polyalkenamer and poly cis-isoprene. Preferably, the first polymer of a composition according to the invention is a polycaprolactone (PCL) polymer, most preferably ε-polycaprolactone. ε-polycaprolactone is particularly preferred because it has a low melting point, shows good moulding properties and has sufficiently long working time before it solidifies in order to allow direct molding on the body. If so desired, the ε-polycaprolactone can be used in a mixture with another thermoplastic, for example thermoplastic polyurethane.

Particularly preferred is a first polymer (in particular ε-polycaprolactone) with a number average molecular weight Mn of at least 10.000 g/mole, more preferably at least 20.000 g/mole, most preferably at least 40.000 g/mole, in particular at least 50.000 g/mole. The first polymer of the invention will generally have a number average molecular weight Mn of maximum 100.000 g/mole, preferably maximum 90.000 g/mole. A first polymer with a molecular weight above 100.000 g/mole may lead to slower crystallisation rates and reduce the moldability of the thermoplastic material in the molten state, thereby compromising the ability of the material to be shaped in the molten state and adversely affecting patient comfort. However crystallization can be speeded up by adding nucleating agents. PCL with varying molecular weights are commercially available from the company Ingevity under the trade designation CAPA.

A composition according to the invention comprises 30 to 62 wt%, preferably 40 to 55 wt%, of a first polymer. In particular, good results have been obtained with PCL (namely Polycaprolactone CAPA 6500, which has a molecular weight of 50,000 g/mole) .

The molecular weight Mn of a compound can, for example, be determined by gel permeation chromatography (GPC) according to DIN 55672-1, using THF as eluent. Unless otherwise indicated, the molecular weights listed are those that have been determined by GPC.

Suitable fillers for use in the present invention comprise inorganic or organic materials which are insoluble in the polymer when the polymer is in the molten phase and which have a spherical morphology such as spheres and bubbles. A spherical filler preferably has a shape factor of below 3, preferably below 2 wherein the term "shape factor" refers to the ratio between the size of the largest dimension axis of the filler particle, and the size of the smallest dimension axis of the filler particle. The shape factor is generally between 1 and 3, preferably between 1 and 2 and most preferably about 1. Such type of fillers provide isotropic properties in extrusion processing. Further with spherical fillers a more stable flow during extrusion is obtained leading to an easier production process.

Although the fillers may have a solid, porous, or hollow structure, a solid structure is preferred, such as solid glass spheres, due to the fact that the shape thereof remains in the thermoplastic composite material whereas shell-type spherical fillers such as hollow bubbles may break during the process of manufacturing the composite material, particularly during an extrusion process.

Preferably, the spherical filler has a diameter size of less than 120 µm, preferably less than 100 µm, more preferably between 40 and 80 µm. Larger sizes generally results in higher pressure during extrusion, particularly when a fine sieve is used to eliminate potential dirtiness and impurities.

Preferred spherical fillers for use according to the invention are glass balloons or glass beads.

A composition according to the invention comprises 15 to 35 wt%, preferably 22-32 wt%, more preferably 25 to 30 wt%, of a spherical filler. Above 35 wt% a fragile behaviour of the thermoplastic composite is observed: a flat sheet of the thermoplastic composite simply breaks by bending at 90°. Fillers agglomeration as well as the absence of chemical bonding between the matrix and the filler contributes to such weak properties. Below 15 wt% not enough strength and stiffness is obtained. In particular, good results have been obtained with glass beads such as Spheriglass 2530 available from Potters Industries, which have a diameter of between 2.8 and 100 µm.

In some embodiments, a composition according to the invention may comprise besides the spherical filler also another type of filler (such as nanoclay) present in a limited amount, such as an amount of below 10 wt%, preferably below 9%, more preferably below 5 wt% of the total composition.

An elastomeric material as comprised in a composition according to the invention can be any material exhibiting elastic or rubber-like properties. Said elastomeric material preferably has a shore A hardness of up to 92, preferably from 30 to 80 and is preferably compatible with the first polymer (e.g. PCL). Suitable elastomeric materials for use according to the present invention include thermoplastic elastomers (TPE), thermoplastic polyurethane (TPU) and so-called thoughened polymers (binary mixtures of rubber and plastics such as polystyrene-butadiene or polystyrene-isoprene copolymers, generally with a styrene content of up to 60 wt%, preferably between 15 and 50 wt%, more preferably between 30 and 35 wt%, wherein the styrene phase is partially miscible with PCL and the rubber phase (butadiene or isoprene) improves the toughness (comfort)).

The elastomeric material preferably has a molecular weight above 100 000 g/mol, preferably above 150 000 g/mol, more preferably about 170,000 g/mol. The viscosity of such elastomeric material expressed as Melt Flow Rate (200°C, 5 kg) measured according to standard ISO1133 is preferably above 1 g/10 min, preferably above 5 g/10 min, more preferably above 10 g/10 min although higher viscosity elastomeric materials having a Melt Flow Rate below 1 g/10 min can be used as well. The Melt Flow Rate is a measure of the ease of flow of the melt of a thermoplastic polymer. It is defined as the mass of polymer, in grams, flowing in ten minutes through a capillary of a specific diameter and length by a pressure applied via prescribed alternative gravimetric weights for alternative prescribed temperatures. Melt Flow Rate is inversely proportional to viscosity of the melt at the conditions of the test. In some embodiments, the elastomeric material may contain viscosity reducing agents such as mineral oils. The Melt Flow Rate (200°C, 5 kg) of such a component may be in the range of 20 to 40 g/10 min.

A composition according to the invention comprises 4 to 20 wt%, preferably 6 to 15 wt%, most preferably 6 to 10 wt%, of such an elastomeric material. Lower amounts of elastomeric material results in high material stretch and low stability during heating and shaping of the composite on the patient. Higher amounts of elastomeric material results in difficulties to shape the composite at melting temperature (generally 65°C) as the composite will be too stiff at activation temperature and too soft at room temperature (during use). In case a low viscosity elastomeric material is used, as specified above, amounts of up to 15 wt% can be used. With higher viscosity elastomeric materials (such as those having a PFR of below 1 g/10 min) generally lower amounts have to be used (up to 10 wt%).

In particular, good results have been obtained with polystyrene-butadiene polymer (namely Kraton D1101AS or Kraton D4153 ES (containing mineral oils to reduce viscosity)).

Optionally a second polymer is comprised in a composition according to the invention. Said second polymer is a polymer with a higher bending modulus than the first polymer, generally a so-called reinforcing thermoplastic. Hence in the case the first polymer is PCL the second polymer should preferably have a bending modulus of at least 800 MPa (measured according to standard ASTM D790). Examples of polymers with such bending moduli can be selected from the family of polyesters, polyolefins, or polyamides. A preferred second polymer as comprised in a composition according to the invention is a polyolefin such as polypropylene (PP), or compositions based on polypropylene. Such second polymers offer physical retention, which makes the resulting product to be easily deformable and shapable without being too flowable.

If such second polymer is present in the composition according to the invention then the amount thereof generally ranges between 2 to 20 wt%, preferably between 5 to 15 wt%. Below 5 wt% in general the bending modulus will not be sufficiently high and the shape not maintained in case there is limited amount of elastomeric material. Above 20 wt% it is not possible to stretch and shape the composite once heated at 65°C as at this temperature said second polymer is not melted nor flowable.

An additive as optionally comprised in a composition according to the invention, may be any of the standard additives like pigments, antioxidants, thickeners, processing aids, stabilizers, plasticizers, lubricants, antistatic agents, nucleating agents, flow improvers.

A composition according to the invention comprises 0 to 7 wt%, preferably 4 to 7 wt%, most preferably 5.5 to 6 wt%, of total additives. Preferably a masterbatch of all additives can be prepared and added to the other ingredients of the composition. The amount of each individual additive present in the composition is preferably between 0.1 and 4 wt %, more preferably between 0.5 and 2 %.

A composition according to the invention may comprise between 0.1 and 0.5 wt%, preferably 0.3 wt%, of nucleating agent. A composition according to the invention may comprise between 2.5 and 10 wt% of wax. A composition according to the invention may comprise between 2 and 8 wt%, preferably 4 wt%, of antisticking agent. A composition according to the invention may comprise between 0.5 and 2.5 wt%, preferably 1.5 wt%, of pigment. A composition according to the invention may comprise between 0.05 and 1 wt%, preferably 0.25 wt%, of lubricant. A composition according to the invention may comprise between 0 and 0.5 wt%, preferably 0.05 wt%, of anti-oxidant.

The invention furthermore relates to a thermoplastic composite material produced from the composition according to the invention. Said thermoplastic composite material may be shaped into a sheet, which is suitable for use in immobilisation devices.

For the production of the thermoplastic composite material according to the invention, each technique which may be deemed suitable by the person skilled in the art can be used such as extrusion and injection-molding. Preferably the thermoplastic composite material is manufactured by extrusion, including single-screw and twin-screw extrusion and extrusion-based processes and can be in the form of granules, sheets and filaments, depending of the way of making the customized product. Plastics extrusion is a well-known manufacturing process in which raw plastic is melted and formed into a continuous profile, for example films or sheets. This process starts by feeding plastic material into the barrel of the extruder. The material is gradually melted by the mechanical energy generated by turning screws and by heaters arranged along the barrel. The molten polymer is then forced into a die, which shapes the polymer into a shape that hardens during cooling.

Manufacturing thermoplastic composite material of the present invention by extrusion preferably takes place in a temperature range of between 180 and 200°C. In general all ingredients of the composition apart from the spherical filler are fed at the first open zone of the extruder barrel and mixed by twin-screwing. The spherical filler is preferably fed later into the extruder barrel via a side feeder when the composition is already melted. However feeding all the ingredients into the first zone can also be envisaged.

A further advantage of the present invention lies in the fact that the end-use properties of the thermoplastic composite material are set during the extrusion process and do not require any post-processing step at the solid state. Such a post processing step refers to vulcanisation or crosslinking that is achieved by the exposure of the polymeric composition to an external energy source (e.g. by heating, photon or electron beam irradiation) that leads, in the presence of a chemical crosslinking agent, to the formation of covalent bonds within, between and through the various polymer chains of the composition.

Preferably the thermoplastic material is produced in the form of a sheet. The thickness of the sheet may vary within wide limits. When using the thermoplastic composite material for an immobilization device for immobilization of a body part, the thickness will generally vary from 1 to 4 mm, preferably 2.4 to 3.2 mm. Often, a thickness of about 3.2 mm is used. The skilled person will ensure in the selection of the suitable thickness that the thickness is sufficiently large to satisfy the requirements for the intended application. For immobilization devices, the thickness is usually chosen such that the flexural modulus and strength end up within the required limits for that application, and that the cooling time of the composite material after being heated to its melting or softening temperature, is sufficiently long to allow forming at/around the body part to be immobilized.

The thermoplastic sheet of the present invention may be perforated or not. The method of applying such perforations to thermoplastic sheets for use in immobilization devices is well known to the skilled person. In general, in view of optimizing the time available for moulding of the template, the degree of perforation of the thermoplastic sheet will vary between 1 and 25 %, wherein % means the % of the surface of the thermoplastic sheet occupied by perforations, in relation to the total surface of the thermoplastic sheet.

According to the invention, a sheet-shaped thermoplastic composite material is used for producing an immobilization device for immobilization of a body part. The material of the present invention is particularly useful in the area of physical rehabilitation immobilisation such as orthosis and splints. Physical therapists, occupational therapists, hand therapists and orthotists soften the thermoplastic material in hot water and then mold it directly to the patients affected body part, creating a form that closely matches the anatomical contours of the patient's affected body part. The orthosis or splint is used to either immobilize the body part to allow for proper healing, to prevent a certain undesirable motion, or to promote a certain desirable motion.

A thermoplastic composite material according to the invention has a high rigidity at room temperature. Therefore, it is particularly suitable for physical rehabilitation application where a high rigidity is required such as anti-spasticity orthosis, dorsal blocking orthosis, wrist cock-up, short opponens, or finger based, digit extension orthoses *status post* Dupuytren's release and resting splints. Despite the relatively high rigidity of the thermoplastic composite material, the orthoses and splint made with it are highly comfortable.

At high temperatures such as temperatures of around 65°C, a thermoplastic composite material according to the invention is in a molten state, providing a high drape behavior. In this molten state, the material is easy to shape or mold around a body part resulting in a good conformity around the body part.

Thermoplastic composite materials according to the invention generally have a drape of below 5 cm, preferably below 4 cm. Preferably the drape is at least 0.5 cm, preferably at least 1 cm. To assess drape, a test specimen is cut to a sheet having the dimensions 10cm x 2.5 cm. A line is drawn with a marker in the middle of the test specimen, 5 cm from the side. Hereafter, the test specimen is heated in a water bath at activation temperature and time (in most cases 65°C for 3 min). After heating, the test specimen is put on a metal pin (Æ=2.4mm), making sure the test specimen does not touch anything and is positioned exactly in the middle. The drawn line should be corresponding with the metal pin. Hereafter the test specimen is released. While the test specimen cools down but is still warm, it will bend due to gravimetrical forces. Once the specimen is completely cooled down, the distance between the two 'legs' of the sample is measured (cm) as an indication of the drape.

Thermoplastic composite materials according to the invention generally have a Resistance to Stress (RTS) of below 35 cm, preferably in the range 15.5 to 35 cm, more preferably 15.5 to 24 cm. RTS is a method to estimate the Resistance To Stretch of the sheet after activation (i.e. heating at 65°C during 3 min) by: (a) cutting a sample with standard dimensions from the extruded sheet, (b) heating the sample in hot water (65 °C) for 3 min., (c) taking out the sample from the hot water and securing one end of the sample with a clamp and providing the other end with a standard weight (135 g), (d) stretching the molten sample under the force of gravity and cooling to room temperature, (e) the value of the length of the stretched sample (cm) is the value of the RTS (cm), (f), the shorter the stretched sample, the higher the resistance to stretch is.

Preferably thermoplastic composite materials according to the invention have a bending modulus of at least equal or higher than the first polymer. So in case the first polymer is polycaprolactone the bending modulus is preferably at least 400 MPa, more preferably at least 500 MPa. The bending modulus is measured according to standard ASTM D790.

Due to the specific ratios of different compounds in the thermoplastic composite material according to the invention, some physical retention is offered so that in the molten state, the material does not fully flow while it can still be easily deformed, shaped and re-shaped for eventual corrections or modification necessary during the patient's treatment. At room temperature the material has sufficient strength to ensure good immobilisation of the body member. Therefore, a sheet of a thermoplastic composite material according to the invention is particularly suitable for use in a immobilization device such as a split or orthosis.

For the comfort of the patient, it is important that the temperature at which the material is shaped or moulded, is less than 90°C, preferably less than 80°C, more preferably less than 75°C, most preferably between 65 and 70°C.

A thermoplastic composite material according to the invention does not cooldown quickly after heating which offers the therapist enough time to re-shape and finish the orthosis. In particular, the inventors have observed that the period of time which remains available for the molding of the thermoplastic material, may be at least 1 minute, in particular at least 1.5 minutes or at least 2 minutes, sometimes more than 2.5 minutes. In general, the molding time available, will not be longer than 15 minutes, preferably the available molding time will not be longer than 12.5 minutes, in particular it will not be longer than 10 minutes or maximum 5 minutes as this would reduce the comfort to the patient due to an increasing cooling and waiting time.

A thermoplastic composite material according to the invention can be coated with a thin layer of a non-sticky coating. Such coatings provide a good balance of residual stickiness as they provide on the one hand a slightly tacky surface which is important to keep material in place when fabricating the orthosis, so it does not move around while on the other hand it does not stick to the patient skin. A suitable non-sticky coating is for example a two component acrylic based coating.

Another feature of a thermoplastic composite material according to the invention is that the material has enough residual stickiness so that if the material is used in a immobilization device, the edges of said device are easy to finish. Due to the residual stickiness, the edges roll down easily and adhere well. Additionally, the edges are easy to trim.

The invention is illustrated by but not limited to the following Examples.

### Example 1 (Comparative example with other types of filler):

A thermoplastic sheet of 3.2 mm thickness was produced by extrusion containing the ingredients as indicated in table 1 below.

The ingredients were fed in zone 1 of ZSK twin screw extruder (Coperion) using two gravimetric feeders. The extrusion temperature was set to 150-160°C. The screw speed was set at 200 rpm. In sample 5 the filler was fed into the extruder barrel through a separate feeder. In all other samples all ingredients were fed into the first zone all at once. Samples were extruded on Tyson extruder (production level) with only flat die with a width of 120-150 cm.

The following properties were measured on the obtained sheet: RTS, drape and bending modulus all according to the methods described above. The results are indicated in Table 1.

From table 1 it can be concluded that the use of other types of fillers than the spherical ones according to the present invention do not provide satisfactory properties in terms of drape behaviour and/or rigidity of the obtained thermoplastic sheets. Further the properties of the samples containing short glass fibers (samples 4 and 5) differ substantially depending on the direction in which the properties were measured (extrusion direction or cross direction).

### Example 2 (Using spherical fillers according to the invention):

A thermoplastic sheet of 3.2 mm thickness was produced by extrusion containing the ingredients as indicated in table 2 below according to the same process as in Example 1. In sample 9 the filler was fed into the extruder barrel through a separate feeder. In all other samples all ingredients were fed into the first zone all at once.

The following properties were measured on the obtained sheet: RTS, drape and bending modulus all according to the methods described above. The results are indicated in Table 2.

From table 2 it can be concluded that compositions according to the invention containing spherical fillers provide thermoplastic sheet having satisfactory properties on rigidity, drape and remains elastomeric in heated state for a sufficient period of time. Further the properties were measured in both directions (extrusion and cross direction) and found to be similar which present an advantage vis-à-vis short glass fibers.

**Table 1**

| Sample No. | | **1** | **2** | **3(**)** | **4(*)** | **5** | **6** |
|---|---|---|---|---|---|---|---|
| **Component** | **/n° formulation** | Wt% | Wt% | Wt% | wt% | wt% | Wt % |
| Polycaprolactone | CAPA 6500 | 59.19 | 59.19 | 58.2 | 58.2 | 58.2 | 58.2 |
| Mica | Mica Mun 85 | 22.51 | 0 | 0 | 0 | 0 | 0 |
| Grinded silica | MicroSilica, Silverbond 500 | 0 | 22.51 | 0 | 0 | 0 | 0 |
| Glass fibers: short | ThermoFlow^{®} 675 | 0 | 0 | 21 | 21 | 0 | 0 |
| Glass fibers: milled | LANXESS MF7904 | 0 | 0 | 0 | 0 | 21 | 0 |
| Glass bubbles | Glass S60HS | 0 | 0 | 0 | 0 | 0 | 21 |
| Styrenic Block Copolymers | Kraton D4153 | 12.5 | 12.5 | 15 | 15 | 15 | 15 |
| Antisticking MB | | 4 | 4 | 4 | 4 | 4 | 4 |
| White pigment | | 1.5 | 1.5 | 1.5 | 1.5 | 1.5 | 1.5 |
| Lubricant | | 0.2 | 0.2 | 0.2 | 0.2 | 0.2 | 0.2 |
| Lubricant | | 0.05 | 0.05 | 0.05 | 0.05 | 0.05 | 0.05 |
| Antioxidant | | 0.05 | 0.05 | 0.05 | 0.05 | 0.05 | 0.05 |
| **Sum Components** | | **100.00** | **100.00** | **100.00** | **100.00** | **100.00** | **100.00** |
| **Objective measurements** | | | | | | | |
| | RTS (135g) | 15.5 | 17 | 21 | 16 | 16 | 15.0 |
| | Sec. Mod. (MPa) | 355 | 305 | 1490 | 392 | 269 | 275.0 |
| Max. Load (N) | | 6.82 | 6.04 | 35.1 | 10.1 | 4.92 | 5.4 |
| Drape (cm) | | 5.5 | 4.2-5.3 | 2.5 | 7.5 | 2.6 | 4.0 |

| | | | | | | | |
|---|---|---|---|---|---|---|---|
| (*) properties measured in cross direction (**) properties measured in extrusion direction | | | | | | | |

**Table 2**

| Sample No. | | **7** | **8** | **9** |
|---|---|---|---|---|
| **Component** | **/n° formulation** | Wt % | Wt % | Wt % |
| Polycaprolactone | CAPA 6500 | 60.2 | 55.2 | 41.2 |
| Glass spheres | Spheriglass 2530 | **26** | **26** | **30** |
| Styrenic Block Copolymer | Kraton D1101AS | 8 | 8 | 8 |
| PP copolymer (second polymer) | Polybatch RTP 1097 | 0 | 5 | 15 |
| Antisticking MB | | 4 | 4 | 4 |
| White pigment | | 1.5 | 1.5 | 1.5 |
| Lubricant | | 0.2 | 0.2 | 0.2 |
| Lubricant | | 0.05 | 0.05 | 0.05 |
| Antioxidant | | 0.05 | 0.05 | 0.05 |
| **Sum Components** | | **100.00** | **100.00** | **100.00** |
| | | | | |
| **Objective measurements** | RTS (135g) | 16.5 | 15.5 | 24.0 |
| | Sec. Mod. (MPa) | 469.4 | 512 | 650 |
| | Max. Load (N) | 9.9 | 10.5 | 17.5 |
| | Drape (cm) | 2.4 | 1.8 | 1.3 |

## Claims

1. A composition comprising, based on total weight of the composition,
a. 30 to 62 wt% of a first polymer with a melting temperature below 100°C, preferably of between 40 and 75°C;
b. 15 to 35 wt% of a spherical filler and optionally up to 10 wt% of another type of filler;
c. 4 to 20 wt% of an elastomeric material;
d. optionally, 2 to 20 wt% of a second polymer with a higher bending modulus than the first polymer;
e. 0 to 7 wt% of total additives.

2. The composition according to claim 1, wherein the first polymer is a polycaprolactone (PCL) polymer.

3. The composition according to claim 1 or claim 2, wherein the filler has a shape factor of 1 to 3.

4. The composition according to any one of the preceding claims wherein the filler is solid.

5. The composition according to any one of the preceding claims wherein the spherical filler comprises solid glass spheres and the optional other type of filler comprises nanoclay.

6. The composition according to any one of the preceding claims wherein the elastomeric material has a molecular weight above 100 000 g/mol.

7. The composition according to any of the preceding claims, wherein the elastomeric material is selected from the group consisting of polystyrene-butadiene preferably having a styrene content of 15 to 50 wt%, Thermoplastic Elastomers or Thermoplastic Polyurethanes, preferably the elastomeric material is a polystyrene-butadiene polymer.

8. The composition according to any of the preceding claims, wherein the second polymer has a bending modulus of at least 800 MPa.

9. The composition according to any of the preceding claims, wherein the second polymer is selected from the family of polyester, polyolefins, or polyamide, preferably the second polymer is a polypropylene, preferably comprising filler.

10. The composition according to any of the preceding claims, wherein the one or more additive is selected from the group consisting of a pigment, antioxidant, thickener, processing aid and slipping agent.

11. A thermoplastic composite material made from a composition as defined in any one of claims 1-10.

12. A thermoplastic composite material according to claim 11 which is made by extrusion whereby preferably all of the ingredients of the composition except the spherical filler are fed in a first stage and the spherical filler is added subsequently through a separate inlet.

13. A thermoplastic composite material according to any one of claims 11 to 12 having a drape of 0.5 to 5 cm, a Resistance to Stress of 15.5 to 35 cm and a bending modulus of at least 400 MPa.

14. A thermoplastic composite material according to any one of claims 11 to 13 which is in the form of a sheet, preferably having a thickness in the range 1 to 4 mm.

15. The use of a thermoplastic composite material as defined in any one of claim 11 to 14, for the manufacture of an immobilization device for immobilizing at least a portion of a body part.

16. An immobilization device for immobilizing at least a portion of a body part, wherein the immobilisation device comprises a sheet of a thermoplastic composite material as defined in claim 14, wherein the sheet of the thermoplastic composite material is shaped to conform to the body part to be immobilized.
